# EUROPÄISCHE PATENTSCHRIFT

(11) **EP 3 669 846 B1**
(45) Veröffentlichungstag und Bekanntmachung des Hinweises auf die Patenterteilung: **30.08.2023**
(21) Anmeldenummer: 19216931.6
(22) Anmeldetag: 17.12.2019
(51) Int. Cl.: A61F 15/00, B65D 83/08

(54) **PFLASTERSPENDER**
PLASTER DISPENSER
DISTRIBUTEUR DE PANSEMENT

(30) Priorität: 20.12.2018 DE 102018222639; 20.12.2018 DE 102018222647
(43) Veröffentlichungstag der Anmeldung: 24.06.2020
(73) Patentinhaber: Wero GmbH & Co. KG, 65232 Taunusstein (DE)
(72) Erfinder: AUBELE, Sebastian, 65232 Taunusstein (DE)
(74) Vertreter: BRP Renaud & Partner mbB Rechtsanwälte Patentanwälte Steuerberater

(56) Entgegenhaltungen:
- EP-A2- 0 948 949
- DE-A1- 2 849 680
- DE-T2-602005 006 374
- US-A1- 2012 292 216

## Beschreibung

Die Erfindung betrifft einen Pflasterspender mit einem Spendergehäuse und wenigstens einer in dem Spendergehäuse angeordneten Pflasternachfülleinheit nach dem Oberbegriff des Anspruchs 1.

Pflasterspender sind aus dem Stand der Technik bereits bekannt. Üblicherweise weist ein Pflasterspender ein Spendergehäuse auf, in dem wenigstens eine Pflasternachfülleinheit angeordnet ist. In der jeweiligen Pflasternachfülleinheit ist ein Pflasterbündel mit mehreren Einzelpflastern angeordnet, die bei Bedarf einzeln aus der Pflasternachfülleinheit entnommen werden können. Sind die Einzelpflaster in dem Pflasterbündel verbraucht, so kann die Pflasternachfülleinheit ersetzt werden. Der Pflasterspender ist dabei üblicherweise an einer Wandung an einem frei zugänglichen Ort befestigt, damit Nutzer einen freien Zugang zu dem Pflasterspender haben. Nachteiligerweise kann dabei die Pflasternachfülleinheit auch unberechtigt aus dem Pflasterspender entnommen werden. Zur Lösung des Problems ist aus DE 28 49 680 A1 beispielsweise bekannt, die Pflasternachfülleinheit formschlüssig in dem Spendergehäuse festzulegen. Dadurch kann eine unberechtigte Entnahme der Pflasternachfülleinheit verhindert werden. Auch in der EP 0 948 949 A2 ist ein Pflasterspender offenbart, in dem die Pflasternachfülleinheit formschlüssig in dem Spendergehäuse festgelegt wird.

Die Aufgabe der Erfindung ist es daher, für einen Pflasterspender der gattungsgemäßen Art eine verbesserte oder zumindest alternative Ausführungsform anzugeben, bei der die beschriebenen Nachteile überwunden werden.

Diese Aufgabe wird erfindungsgemäß durch den Gegenstand des unabhängigen Anspruchs 1 gelöst. Vorteilhafte Ausführungsformen sind Gegenstand der abhängigen Ansprüche.

Ein gattungsgemäßer Pflasterspender weist ein Spendergehäuse und wenigstens einer in dem Spendergehäuse angeordneten Pflasternachfülleinheit, der eine Aufnahmebox und wenigstens ein Pflasterbündel aufweist. Das Pflasterbündel ist dabei in einem Boxinnenraum der Aufnahmebox festgelegt und weist mehrere in Entnahmerichtung aus der Pflasternachfülleinheit entnehmbare Einzelpflaster auf. Die wenigstens eine Pflasternachfülleinheit ist in einem Spenderinnenraum des Spendergehäuses aus diesem in Entnahmerichtung entnehmbar angeordnet. Dabei ist die wenigstens eine Pflasternachfülleinheit in dem Spenderinnenraum durch wenigstens ein formschlüssiges Formschloss lösbar festgelegt. Dabei greifen ein an der Aufnahmebox ausgebildetes erstes Formschlossteil und ein in dem Spenderinnenraum federnd angeordnetes zweites Formschlossteil quer zur Entnahmerichtung formschlüssig ineinander ein. Dadurch ist die Pflasternachfülleinheit aus dem Spenderinnenraum beim geschlossenen Formschloss nicht entnehmbar und beim geöffneten Formschloss entnehmbar. Bei dem geschlossenen Formschloss ist die Pflasternachfülleinheit in dem Spenderinnenraum gegen unberechtigte Entnahme gesichert.

Die Entnahmerichtung erstreckt sich in der vorliegenden Erfindung parallel zur Längsachse der Aufnahmebox und ist aus dem Boxinnenraum der in dem Spendergehäuse festgelegten Pflasternachfülleinheit heraus gerichtet. Zweckgemäß sind in dem wenigstens einen Pflasterbündel der in dem Spendergehäuse festgelegten Pflasternachfülleinheit Längsrichtungen der Einzelpflaster etwa in Entnahmerichtung ausgerichtet, um ein Entnehmen der Einzelpflaster in Entnahmerichtung zu ermöglichen. Es versteht sich von selbst, dass das tatsächliche Entnehmen der Einzelpflaster aus der Pflasternachfülleinheit und der Pflasternachfülleinheit aus dem Spendergehäuse nutzerabhängig ist und in einer von der Entnahmerichtung abweichende Richtung erfolgen kann. In dem erfindungsgemäßen Pflasterspender ist die Pflasternachfülleinheit in dem Spendergehäuse formschlüssig festgelegt, so dass ein unberechtigtes Entnehmen der Pflasternachfülleinheit aus dem Spendergehäuse verhindert ist. Die Einzelpflaster können in dem Pflasterbündel ferner derart festgelegt sein, dass die Einzelpflaster aus dem Pflasterbündel einsatzbereit entnehmbar sind. Beispielsweise kann durch das Herausziehen des Einzelpflasters bereits eine Klebefläche des Einzelpflasters mehr oder weniger freigelegt sein, so dass das Einzelpflaster gleich verwendet werden muss. Ein Herausnehmen auf Vorrat erschwert sich dadurch. Auf diese Weise kann somit auch ein unberechtigtes Entnehmen der Einzelpflaster aus der Pflasternachfülleinheit vorteilhaft verhindert werden. Der Pflasterspender kann dann frei zugänglich bleiben. Der Pflasterspender ist dabei dazu vorgesehen, aufrecht ausgerichtet bzw. nach oben offen an einer Wandung befestigt zu sein. Das Entnehmen der Einzelpflaster kann dann von oben erfolgen. Die Entnahmerichtung erstreckt sich dann von unten nach oben. Die Begriffe "oben" und "unten" beziehen sich dabei auf die Richtung der Erdanziehungskraft.

Erfindungsgemäß ist vorgesehen, dass das erste Formschlossteil durch eine Eingriffsöffnung gebildet ist und das zweite Formschlossteil einen Eingriffsvorsprung bzw. eine Eingriffsnase aufweist. Die Eingriffsöffnung ist dann in einer Außenwandung der Aufnahmebox ausgebildet und die in dem Spenderinnenraum angeordnete Eingriffsnase greift quer zur Entnahmerichtung in die Eingriffsöffnung formschlüssig ein. Zweckgemäß ist die Eingriffsnase federnd, so dass beim Einsetzen der Pflasternachfülleinheit in das Spendergehäuse die Eingriffsnase in die Eingriffsöffnung selbsttätig einfedert und das Formschloss automatisch schließt. Dadurch kann die Nachbestückung des Pflasterspenders vereinfacht werden. Grundsätzlich können die Eingriffsöffnung in dem Spenderinnenraum und die Eingriffsnase in der Außenwandung der Aufnahmebox ausgebildet sein. Da jedoch die Aufnahmebox der Pflasternachfülleinheit üblicherweise aus Papier oder Karton geformt ist, ist die Eingriffsnase an der Außenwandung der Aufnahmebox nur schwer beziehungsweise nur kostenintensiv realisierbar. Aus diesem Grund wird in der vorliegenden Erfindung die oben beschriebene Ausgestaltung des Formschlosses mit der Eingriffsöffnung in der Aufnahmebox bevorzugt.

Zudem ist erfindungsgemäß vorgesehen, dass das zweite Formschlossteil ein zungenartiger und federnder Zungenbereich ist, an dem dann die Eingriffsnase ausgebildet ist. Der Zungenbereich ist dabei an einer den Spenderinnenraum begrenzenden Spenderwand des Spendergehäuses ausgebildet und die Eingriffsnase ist innerhalb des Spenderinnenraums angeordnet. Der Eingriff zwischen der Eingriffsnase und der Eingriffsöffnung ist zudem von außen ohne weitere Mittel nicht lösbar. Ist der Pflasterspender mit der Spenderwand an einer Wandung festgelegt, dann ist der Zungenbereich der Wandung zugewandt angeordnet und das Formschloss kann von außen nicht erreicht und ohne weitere Mittel nicht geöffnet werden. Vorteilhafterweise kann zusätzlich vorgesehen sein, dass die Spenderwand ein von dem Spenderinnenraum abgewandtes Außenteil und ein dem Spenderinnenraum zugewandtes Innenteil aufweist. Der Zungenbereich ist dann an dem Außenteil der Spenderwand ausgebildet und die Eingriffsnase ragt durch eine Aussparung in dem Innenteil der Spenderwand in den Spenderinnenraum hinein. In dem Spenderinnenraum ragt dann die Eingriffsnase bei der eingesetzten Pflasternachfülleinheit in die Eingriffsöffnung der Aufnahmebox hinein, so dass die Pflasternachfülleinheit in dem Spenderinnenraum formschlüssig festgelegt ist. Der Zungenbereich ist dabei von dem Spenderinnenraum durch das Innenteil nahe vollständig abgegrenzt und ist auch aus dem Spenderinnenraum nicht zugänglich. Dadurch kann das Formschloss nicht oder nur mit einem erheblichen Aufwand aus dem Spenderinnenraum geöffnet werden. Ist der Pflasterspender mit der Spenderwand an einer Wandung festgelegt, so sind das Außenteil und der Zungenbereich der Wandung zugewandt und das Formschloss kann - wie oben bereits beschrieben - von außen ohne zusätzliche Mittel nicht geöffnet werden.

Bei einer bevorzugten Ausgestaltung des Pflasterspenders ist vorgesehen, dass das Formschloss einen Formschlüssel aufweist. Der Formschlüssel löst dabei den Eingriff zwischen dem ersten Formschlossteil und dem zweiten Formschlossteil. Das Formschloss ist in dem Pflasterspender zweckgemäß von außen unzugänglich ausgebildet, so dass das Formschloss ausschließlich durch den Formschlüssel geöffnet werden kann. Entsprechend kann die Pflasternachfülleinheit aus dem Spendergehäuse ausschließlich durch berechtigte Personen mit dem Formschlüssel entnommen werden. Um das Formschloss mit dem Formschlüssel zu öffnen, kann das zweite Formschlossteil einen Führungsabschnitt aufweisen. Beim Führen des Formschlüssels in Entnahmerichtung kann dann der Führungsabschnitt des zweiten Formschlossteils an dem Formschlüssel abgleiten und das zweite Formschlossteil dadurch quer zur Entnahmerichtung von dem ersten Formschlossteil hinweg geführt sein, so dass der Eingriff zwischen dem zweiten Formschlossteil und dem ersten Formschlossteil gelöst ist.

Sind das erste Formschlossteil durch die Eingriffsöffnung und das zweite Formschlossteil durch den Zungenbereich mit der Eingriffsnase und die Spenderwand aus dem Außenteil und dem Innenteil wie oben beschrieben gebildet, so kann der Führungsabschnitt an dem Zungenbereich des Außenteils ausgebildet sein. Der Führungsabschnitt des Zungenbereichs kann dabei unter einem Winkel zur Entnahmerichtung ausgerichtet sein, so dass beim Führen des Formschlüssels in Entnahmerichtung der Führungsabschnitt an dem Formschlüssel abgleitet und der Zungenbereich gegen die Federkraft quer zur Entnahmerichtung von dem Innenteil geführt wird. Dabei gleitet die Eingriffsnase aus der Eingriffsöffnung heraus und das Formschloss wird geöffnet. Die Pflasternachfülleinheit kann nun aus dem Spendergehäuse entnommen werden. Da der Formschlüssel beim Öffnen des Formschlosses gegen die Federkraft des Zungenbereichs wirkt, federt der Zungenbereich des Außenteils nach dem Entnehmen des Formschlüssels zurück und die Eingriffsnase ragt durch die Aussparung in dem Innenteil in den Spenderinnenraum hinein. Wird nun die Pflasternachfülleinheit in den Spenderrinnenraum eingesetzt, so kann die Eingriffsnase selbsttätig in die Eingriffsöffnung einfedern und das Formschloss geschlossen werden. Ist der Pflasterspender an einer Wandung festgelegt, so ist der Zungenbereich des Außenteils von dem Spenderinnenraum durch das Innenteil und von außen durch die Wandung abgegrenzt. Das Formschloss kann dadurch nur durch berechtigte Personen mit dem Formschlüssel geöffnet werden.

Um das Führen des Formschlüssels in Entnahmerichtung zu vereinfachen, kann an dem Spendergehäuse für den Formschlüssel eine dem Formschlüssel komplementäre Führungsspur ausgebildet sein. Dabei sind der Formschlüssel in der Führungsspur in Entnahmerichtung und dadurch das zweite Formschlossteils an dem Formschlüssel quer zur Entnahmerichtung führbar. So kann die Führungsspur beispielweise eine längliche Ausnehmung sein, in die eine komplementäre Ausformung an dem Formschlüssel einer Nut-Feder-Verbindung ähnlich eingreift. Denkbar ist auch, dass die Führungsspur eine längliche Ausformung ist und in eine komplementäre Nut des Formschlüssels eingreift. Sind das erste Formschlossteil durch die Eingriffsöffnung und das zweite Formschlossteil durch den Zungenbereich mit der Eingriffsnase und die Spenderwand aus dem Außenteil und dem Innenteil wie oben beschrieben gebildet, kann die Führungsspur an dem Außenteil und/oder an dem Innenteil ausgebildet sein.

Vorteilhafterweise kann vorgesehen sein, dass der Spenderinnenraum quer zur Entnahmerichtung durch einen federnden Gehäuseboden begrenzt ist. Dabei ist beim Führen des Formschlüssels in Entnahmerichtung der an den Gehäuseboden abgestützte Pflasterspender in Entnahmerichtung aus dem Spenderinnenraum verschiebbar. Auf diese Weise kann beim Öffnen des Formschlosses mit dem Formschlüssel der Eingriff zwischen dem Pflasterspender und dem Spendergehäuse gelöst und die Pflasternachfülleinheit aus dem Spenderinnenraum herausgeschoben werden. Auf diese Weise kann das Entnehmen der Pflasternachfülleinheit aus dem Spendergehäuse vereinfacht werden. Ferner kann an dem Verschieben der Pflasternachfülleinheit das Öffnen des Formschlosses erkannt werden. Der Gehäuseboden ist federnd und federt nach dem Entfernen des Formschlüssels selbsttätig zurück.

Bei einer vorteilhaften Ausgestaltung des Pflasterspenders ist vorgesehen, dass das Spendergehäuse ein vorderes Gehäusestück und ein hinteres Gehäusestück aufweist. Das vordere Gehäusestück und das hintere Gehäusestück sind dann aneinander zu dem Spendergehäuse festgelegt. Ferner begrenzt das vordere Gehäusestück zumindest vierseitig den Spenderinnenraum und weist einen schwenkbaren Gehäusedeckel zum Verschließen des Spenderinnenraums auf. Das vordere Gehäusestück und das hintere Gehäusestück können aneinander kraftschlüssig oder formschlüssig oder stoffschlüssig festgelegt sein. In der vorliegenden Erfindung beziehen sich die Begriffe "vorne" und "hinten" auf den an einer Wandung befestigten Pflasterspender. In diesem sind das hintere Gehäusestück der Wandung und das vordere Gehäusestück Nutzern zugewandt angeordnet.

Sind das erste Formschlossteil durch die Eingriffsöffnung und das zweite Formschlossteil durch den Zungenbereich mit der Eingriffsnase und die Spenderwand aus dem Außenteil und dem Innenteil wie oben beschrieben gebildet, so kann das Innenteil der Spenderwand in dem vorderen Gehäusestück integral ausgebildet sein. Ferner kann das Außenteil der Spenderwand zumindest bereichsweise das hintere Gehäusestück bilden und an einem Restteil des hinteren Gehäusestücks form- oder kraftschlüssig lösbar festgelegt sein. Vorteilhafterweise kann zusätzlich vorgesehen sein, dass in dem hinteren Gehäusestück außerhalb des Außenteils wenigstens eine Schraubenöffnung ausgebildet ist. Durch die Schraubenöffnung hindurch ist dann das hintere Gehäusestück an einer Wandung festlegbar. Die wenigstens eine Schraubenöffnung kann ferner mit einer Durchgangsöffnung in dem Innenteil korrespondieren, um das Festlegen des hinteren Gehäusestücks an der Wandung aus dem Spenderinnenraum zu ermöglichen.

Die Durchgangsöffnung und die Schraubenöffnung können durch die in dem Spenderinnenraum festgelegte Pflasternachfülleinheit abgedeckt sein, um ein unberechtigtes Lösen des Pflasterspenders von der Wandung zu verhindern.

Vorteilhafterweise kann vorgesehen sein, dass die Außenwandung der Aufnahmebox aus dem vorderen Gehäusestück in Entnahmerichtung zumindest bereichsweise herausragt. Dabei ist ein Erkennungsbereich der Außenwandung von dem hinteren Gehäusestück abgewandt freigelegt, so dass eine in dem Erkennungsbereich der Außenwandung enthaltene Information für den Nutzer erkennbar ist. Alternativ kann das vordere Gehäusestück zumindest bereichsweise aus einem durchsichtigen Material geformt sein. Dabei ist dann ein Erkennungsbereich der Außenwandung von dem hinteren Gehäusestück abgewandt durch das vordere Gehäusestück sichtbar und dadurch ist eine in dem Erkennungsbereich der Außenwandung aufgebrachte Information für den Nutzer erkennbar. Der Erkennungsbereich kann dabei beispielweise eine Bezeichnung oder ein Verwendungszweck oder eine Marke der Einzelpflastern der jeweiligen Pflasternachfülleinheit enthalten. Sind in dem Pflasterspender mehrere Pflasternachfülleinheiten mit abweichend ausgestalteten - beispielweise mit elastischen und wasserabweisenden - Einzelpflastern angeordnet, so kann der Nutzer dies von außen erkennen. Dadurch kann die Wahl des Einzelpflasters für den Nutzer vereinfacht werden.

Vorteilhafterweise können die Einzelpflaster in dem Pflasterbündel durch Schutzhüllen voneinander getrennt und nach außen geschützt sein. Die jeweilige Schutzhülle kann das jeweilige Einzelpflaster nur bereichsweise umhüllen, so dass das jeweilige Einzelpflaster der Entnahmeseite zugewandt aus der jeweiligen Schutzhülle herausragt. Die Einzelpflaster weisen dabei üblicherweise eine Rückenlage auf, wobei eine Funktionsseite der Rückenlage eine Haftmittelschicht, eine Wundauflage und zwei die Haftmittelschicht und Wundauflage überdeckende Deckfolien aufweist. Die Deckfolien erstrecken sich dabei von den Enden der Rückenlage bis zu der Wundauflage und überlappen einander so, dass die Wundauflage nach außen abgedeckt ist. Um die Einzelpflaster einsatzbereit aus der Schutzhülle entnehmen zu können, kann eine der beiden Deckfolien dann innerhalb der Schutzhülle an dieser festgelegt - beispielweise mit der Schutzhülle verklebt - sein. Um ein Entnehmen der Einzelpflaster zu vereinfachen, können diese gefaltet sein. Dabei liegt die Rückenlage der Funktionsseite abgewandt abschnittsweise auf sich selbst an. Zweckgemäß ragen dann die Einzelpflaster an dem gefalteten Ende aus der Aufnahmebox der Nachfülleinheit heraus.

Vorteilhafterweise kann die Außenwandung der Aufnahmebox quer zur Entnahmerichtung einen rechteckigen Querschnitt aufweisen und einseitig durch eine Abstützwand gebildet sein. Das Pflasterbündel kann dann mit der Abstützwand der Außenwandung der Entnahmeseite gegenüberliegend verspannt sein, so dass die Einzelpflaster sich zu der Entnahmeseite hin quer zur Entnahmerichtung auffächern. Dadurch kann das Entnehmen der Einzelpflaster in Entnahmerichtung erleichtert werden. Die Abstützwand ist also parallel zur Entnahmerichtung ausgerichtet. Dabei kann die Abstützwand an der Spenderwand benachbart bzw. an der Spenderwand anliegend angeordnet und die Eingriffsöffnung in der Abstützwand ausgebildet sein. Alternativ kann die Abstützwand von der Spenderwand abgewandt angeordnet und die Eingriffsöffnung des Formschlosses in einer der Abstützwand gegenüberliegenden Wand ausgebildet sein. Zweckgemäß ist die Abstützwand in beiden Fällen zu der Spenderwand parallel ausgerichtet.

Zusätzlich kann vorgesehen sein, dass innerhalb des Boxinnenraums eine Haltewand der Aufnahmebox und die Abstützwand der Außenwandung eine zu der Entnahmeseite hin offene Aufnahmetasche bilden. Die Haltewand und die Abstützwand laufen der Entnahmeseite gegenüberliegend zusammen, so dass die Aufnahmetasche einen dreieckigen Querschnitt aufweist. Das Pflasterbündel kann dann in der Aufnahmetasche angeordnet und mit der Haltewand und der Abstützwand verspannt sein. Vorteilhafterweise kann durch die Haltewand das Pflasterbündel besser abgestützt werden.

Vorteilhafterweise können die Einzelpflaster des Pflasterbündels an der Entnahmeseite abwechselnd auf einen abweichenden Abstand hinausragen. Dies kann das Entnehmen der Einzelpflaster in Entnahmerichtung deutlich vereinfachen.

Zusammenfassend ist die Pflasternachfülleinheit aus dem Spenderinnenraum des erfindungsgemäßen Pflasterspenders beim geschlossenen Formschloss nicht entnehmbar. Dadurch kann ein unberechtigtes Entnehmen der Pflasternachfülleinheit vorteilhaft verhindert werden.

Weitere wichtige Merkmale und Vorteile der Erfindung ergeben sich aus den Unteransprüchen, aus den Zeichnungen und aus der zugehörigen Figurenbeschreibung anhand der Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungsbeispiele der Erfindung sind in den Zeichnungen dargestellt und werden in der nachfolgenden Beschreibung näher erläutert, wobei sich gleiche Bezugszeichen auf gleiche oder ähnliche oder funktional gleiche Komponenten beziehen.

Es zeigen, jeweils schematisch
- Fig. 1: eine Schnittansicht eines erfindungsgemäßen Pflasterspenders;
- Fig. 2 und 3: eine Ansicht und eine Explosionsansicht eines Spendergehäuses des erfindungsgemäßen Pflasterspenders;
- Fig. 4: eine Ansicht eines vorderen Gehäusestücks des Spendergehäuses;
- Fig. 5: eine Ansicht eines hinteren Gehäusestücks des Spendergehäuses ohne ein Außenteil;
- Fig. 6: eine Ansicht des Außenteils des hinteren Gehäusestücks;
- Fig. 7 bis 10: Schnittansichten des erfindungsgemäßen Pflasterspenders beim Entnehmen einer Pflasternachfülleinheit;
- Fig. 11 und 12: Ansichten eines Formschlüssels zum Entnehmen der Pflasternachfülleinheit.

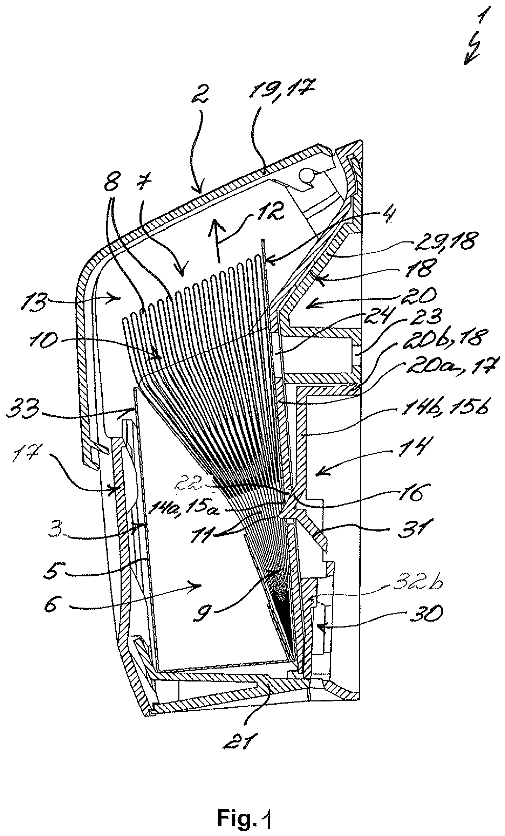

Fig. 1 zeigt eine Schnittansicht eines erfindungsgemäßen Pflasterspenders 1. Der Pflasterspender 1 weist ein Spendergehäuse 2 und zwei Pflasternachfülleinheiten 3 - hier nur einer zu sehen - auf, die nebeneinander in dem Spendergehäuse 2 angeordnet sind. Die Pflasternachfülleinheit 3 weist dabei eine Aufnahmebox 4 auf, deren Außenwandung 5 einen Boxinnenraum 6 nach außen abgrenzt. In dem Boxinnenraum 6 ist ein Pflasterbündel 7 mit mehreren Einzelpflaster 8 in einer Aufnahmetasche 9 angeordnet, so dass die Einzelpflaster 8 zu einer offenen Entnahmeseite 10 der Aufnahmebox 4 auffächern. Dadurch können die Einzelpflaster 8 an der Entnahmeseite 10 vereinfacht aus der Pflasternachfülleinheit 3 entnommen werden. Die Einzelpflaster 8 sind in dem Pflasterbündel 7 durch Schutzhüllen 11 voneinander getrennt und nach außen geschützt. Die Einzelpflaster 8 ragen dabei aus den Schutzhüllen 11 an der Entnahmeseite 10 heraus und können dadurch einsatzbereit aus dem Pflasterbündel 7 in Entnahmerichtung 12 entnommen werden. Zweckgemäß sind in dem Pflasterbündel 7 Längsrichtungen der Einzelpflaster 8 in einer der Entnahmerichtung 12 parallelen Ebene ausgerichtet, um ein Entnehmen der Einzelpflaster 8 in Entnahmerichtung 12 zu vereinfachen. Die Schutzhüllen 11 stehen der Entnahmeseite 10 gegenüberliegend von den Einzelpflastern 8 ab und sind in diesem abstehenden Bereich in der Aufnahmetasche 9 festgelegt. Auf diese Weise kann das Pflasterbündel 7 aus der Aufnahmebox 4 nicht entnommen werden.

Die Pflasternachfülleinheit 3 ist in einem Spenderinnenraum 13 des Spendergehäuses 2 angeordnet und in Entnahmerichtung 12 aus diesem entnehmbar. Die Entnahmerichtung 12 erstreckt sich in der vorliegenden Erfindung parallel zur Längsachse der Aufnahmebox 4 und ist an der Entnahmeseite 10 aus dem Boxinnenraum 6 der in dem Spenderinnraum 13 eingesetzten Pflasternachfülleinheit 3 heraus gerichtet. Sowohl die Einzelpflaster 8 aus der Pflasternachfülleinheit 3 als auch die jeweilige Pflasternachfülleinheit 3 aus dem Spendergehäuse 2 werden erfindungsgemäß in die Entnahmerichtung 12 entnommen. Es versteht sich von selbst, dass das tatsächliche Entnehmen der Einzelpflaster 8 und der jeweiligen Pflasternachfülleinheit 3 aus dem Spendergehäuse 2 nutzerabhängig ist und in einer von der Entnahmerichtung 12 abweichende Richtung erfolgen kann. Ist der Pflasterspender 1 aufrecht ausgerichtet, so entspricht dann das Entnehmen in Entnahmerichtung 12 einem Entnehmen nach oben. Abweichend zu der Entnahmerichtung 12 können die Einzelpflaster 8 dann rechts nach oben; links nach oben oder nach vorne entnommen werden.

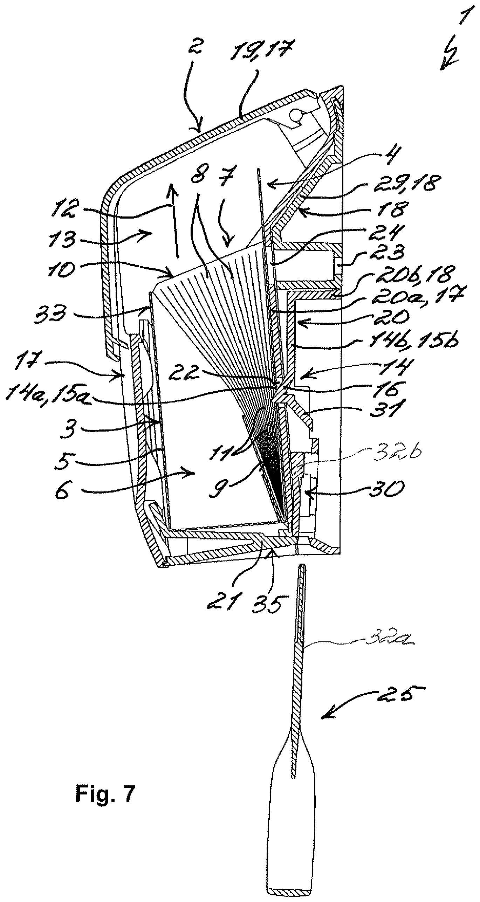

Um ein unberechtigtes Entnehmen der jeweiligen Pflasternachfülleinheit 3 aus dem Spendergehäuse 2 zu verhindern, ist die jeweilige Pflasternachfülleinheit 3 in dem Spenderinnenraum 13 durch ein formschlüssiges Formschloss 14 lösbar festgelegt. Das Formschloss 14 umfasst dabei ein erstes Formschlossteil 14a und ein zweites Formschlossteil 14b, die quer zur Entnahmerichtung 12 ineinander greifen. In diesem Ausführungsbeispiel ist das erste Formschlossteil 14a durch eine Eingriffsöffnung 15a in der Außenwandung 5 der Aufnahmebox 4 gebildet. Das zweite Formschlossteil 14b ist in diesem Ausführungsbeispiel durch einen zungenartigen federnden Zungenbereich 15b mit einer Eingriffsnase 16 gebildet. Wie hier gezeigt, greift die Eingriffsnase 16 in die Eingriffsöffnung 15a quer zur Entnahmerichtung 12 ein und das Formschloss 14 ist geschlossen. Das Öffnen des Formschlosses 14 wird im Folgenden anhand Fig. 7 bis Fig. 10 näher erläutert.

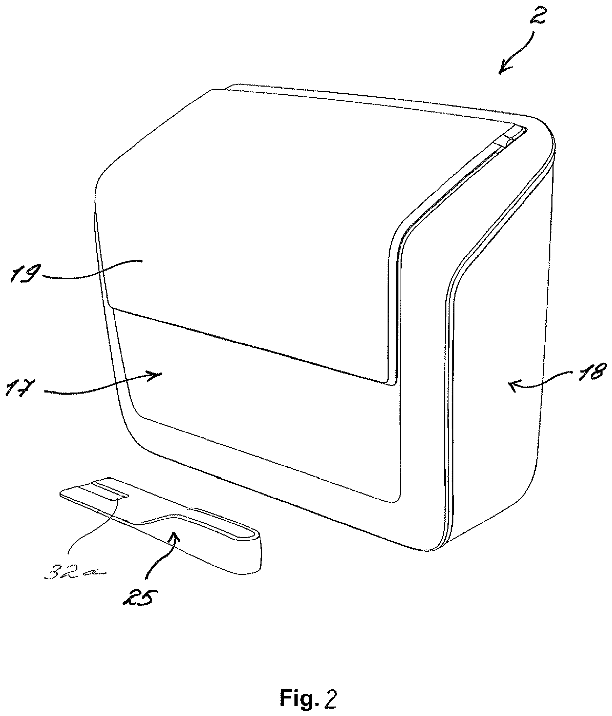

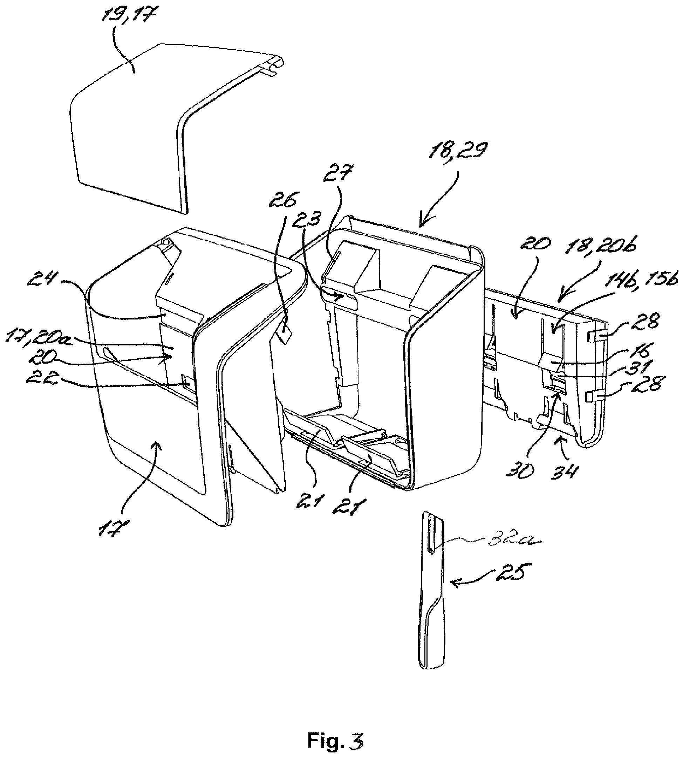

Das Spendergehäuse 2 ist mehrteilig und weist ein vorderes Gehäusestück 17 und ein hinteres Gehäusestück 18 auf. Das vordere Gehäusestück 17 und das hintere Gehäusestück 18 sind aneinander zu dem Spendergehäuse 2 festgelegt, wie im Folgenden anhand Fig. 3 näher erläutert wird. Das vordere Gehäusestück 17 grenzt dabei vierseitig den Spenderinnenraum 13 ab und weist einen schwenkbaren Gehäusedeckel 19 zum Verschließen des Spenderinnenraums 13 auf. Vorteilhafterweise ragt die Außenwandung 5 an dem vorderen Gehäusestück 17 in Entnahmerichtung 12 bereichsweise heraus, so dass ein Erkennungsbereich 33 der Außenwandung 5 von dem hinteren Gehäusestück 18 abgewandt freigelegt ist. Der Erkennungsbereich 33 der Außenwandung 5 kann dann Informationen - beispielweise eine Bezeichnung oder ein Verwendungszweck oder eine Marke - zu den Einzelpflaster 8 der jeweiligen Pflasternachfülleinheit 3 enthalten. Sind in dem Pflasterspender 1 zwei Pflasternachfülleinheiten 3 mit abweichend ausgestalteten Einzelpflastern 8 angeordnet, so kann der Nutzer dies erkennen. Alternativ kann das vordere Gehäusestück 17 zumindest bereichsweise aus einem durchsichtigen Material geformt sein. Dann muss der Erkennungsbereich 33 der Außenwandung 5 nicht freigelegt sein und Informationen in dem Erkennungsbereich 33 sind durch das Material des vorderen Gehäusestücks 17 hindurch sichtbar. Das hintere Gehäusestück 18 bildet einen Gehäuseboden 21 in dem Spenderinnenraum 13, an den sich die jeweilige Pflasternachfülleinheit 3 abstützt. Das vordere Gehäusestück 17 und das hintere Gehäusestück 18 bilden eine Spenderwand 20, die ein Innenteil 20a und ein Außenteil 20b umfasst. Das Innenteil 20a ist integral an dem vorderen Gehäusestück 17 beziehungsweise einstückig mit diesem ausgebildet und das Außenteil 20b bildet bereichsweise das hintere Gehäusestück 18. Das Außenteil 20b ist an einem Restteil 29 des hinteren Gehäusestücks 18 kraftschlüssig festgelegt. Der Aufbau des Spendergehäuses 2 ist in Fig. 2 bis Fig. 6 im Einzelnen gezeigt.

Das zweite Formschlossteil 14b beziehungsweise der Zungenbereich 15b ist an dem Außenteil 20b der Spenderwand 20 ausgebildet und ist von dem Spenderinnenraum 13 nahe vollständig von dem Innenteil 20a abgegrenzt. Die Eingriffsnase 16 ragt dabei durch eine Aussparung 22 in dem Innenteil 20a in den Spenderrinnenraum 13 hinein und greift in das zweite Formschlossteil 14b beziehungsweise in die Eingriffsöffnung 15a ein. Der Zungenbereich 15b ist aus dem Spenderinnenraum 13 nicht zugänglich, so dass das Formschloss 14 nicht oder nur mit einem erheblichen Aufwand aus dem Spenderinnenraum 13 geöffnet werden kann.

Der Pflasterspender 1 wird zweckgemäß mit der Spenderwand 20 an einer Wandung - hier nicht gezeigt - befestigt, so dass der Zungenbereich 15b auch von außen unzugänglich bleibt. Dazu sind in dem hinteren Gehäusestück 18 außerhalb des Außenteils 20b Schraubenöffnungen 23 - hier nur eine zu sehen - ausgebildet. Durch die jeweilige Schraubenöffnung 23 hindurch kann dann das hintere Gehäusestück 18 an der Wandung festgelegt sein. Die jeweilige SchraubenÖffnung 23 korrespondiert mit einer Durchgangsöffnung 24 in dem Innenteil 20a, um das Festlegen des hinteren Gehäusestücks 18 an der Wandung und das Lösen des hinteren Gehäusestücks 18 von der Wandung aus dem Spenderinnenraum 13 zu ermöglichen. Die Durchgangsöffnung 24 und die Schraubenöffnung 23 sind dabei in dem Spenderinnenraum 13 durch die Außenwandung 5 der Aufnahmebox 4 abgedeckt, um ein unberechtigtes Lösen des Pflasterspenders 1 von der Wandung zu verhindern. Ist nun der Pflasterspender 1 mit der Spenderwand 20 an der Wandung festgelegt, so sind das Außenteil 20b und der Zungenbereich 15b der Wandung zugewandt und das Formschloss 14 kann auch von außen ohne zusätzliche Mittel nicht geöffnet werden. Das Öffnen des Formschlosses 14 wird im Folgenden anhand Fig. 7 bis Fig. 10 näher erläutert.

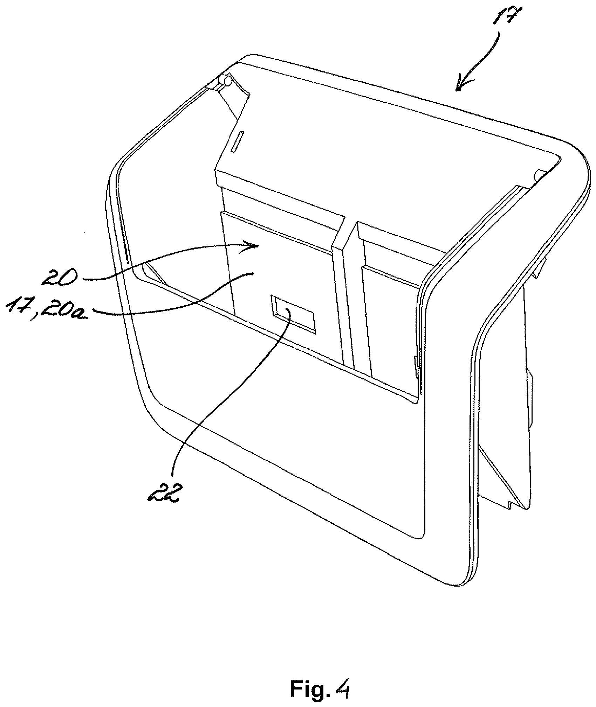

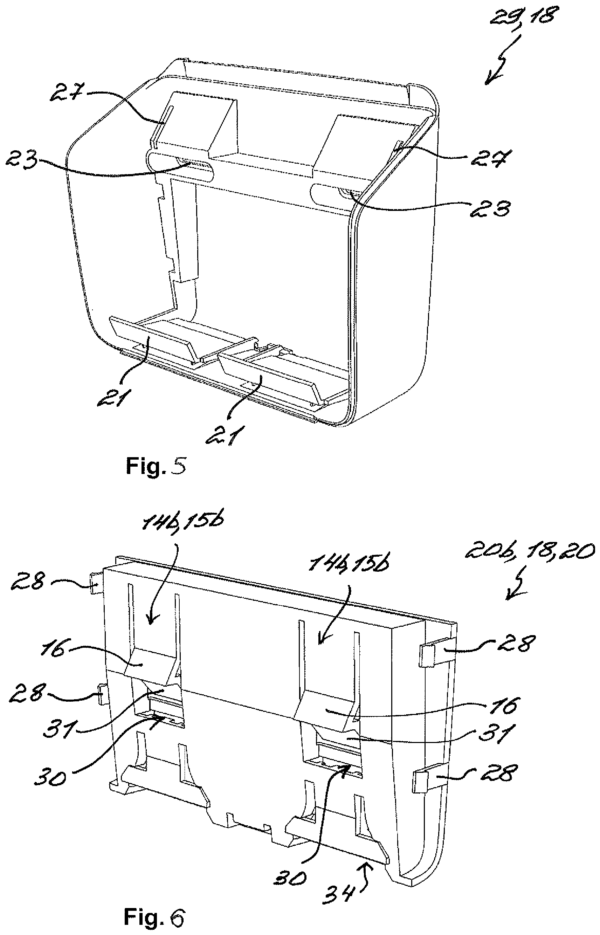

Fig. 2 zeigt eine Ansicht des Spendergehäuses 2 des Pflasterspenders 1. Neben dem Spendergehäuse 2 ist ein Formschlüssel 25 abgebildet, der zum Öffnen des Formschlosses 14 nach Fig. 7 bis Fig. 10 einsetzbar ist. Fig. 3 zeigt eine Explosionsansicht des Spendergehäuses 2. An dem vorderen Gehäusestück 17 sind Klemmhaken 26 und an dem hinteren Gehäusestück 18 sind Klemmöffnungen 27 sichtbar, durch die das vordere Gehäusestück 17 an dem hinteren Gehäusestück 18 klemmend festlegbar ist. Ferner sind an dem Außenteil 20b Haken 28 sichtbar, durch die das Außenteil 20b an dem Restteil 29 des hinteren Gehäusestücks 18 kraftschlüssig festgelegt ist. Fig. 4 zeigt eine Ansicht des vorderen Gehäusestücks 17 des erfindungsgemäßen Pflasterspenders 1. Fig. 5 zeigt eine Ansicht des Restteils 29 des hinteren Gehäusestücks 18. Fig. 6 zeigt eine Ansicht des Außenteils 20b des hinteren Gehäusestücks18. In Fig. 2 bis Fig. 6 ist erkennbar, dass der hier gezeigte Pflasterspender 1 für zwei nebeneinander angeordnete Pflasternachfülleinheiten 3 ausgebildet ist. Für jede der Pflasternachfülleinheiten 3 ist in dem Spendergehäuse 2 jeweils ein Formschloss 14 ausgebildet. Die beiden Formschlösse 14 sind dabei identisch und können mit dem einzigen Formschlüssel 25 geöffnet werden.

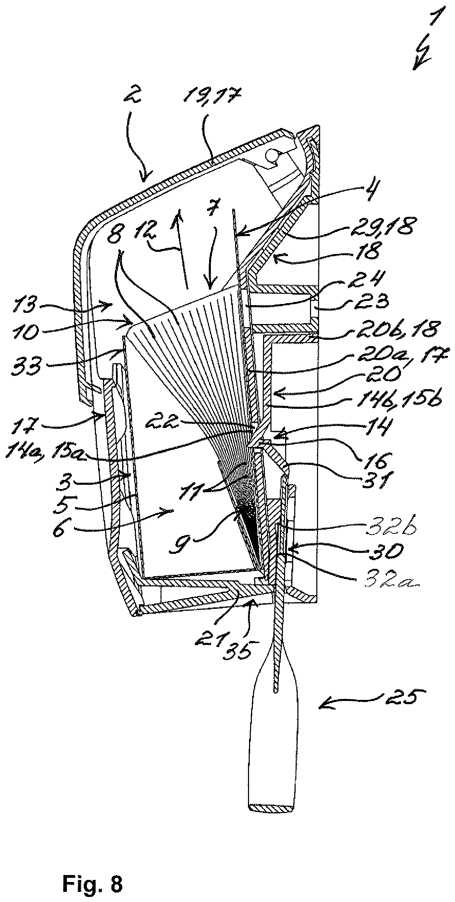

Fig. 7 bis Fig. 10 zeigen Schnittansichten des Pflasterspenders 1 beim Entnehmen der Pflasternachfülleinheit 3 aus dem Spendergehäuse 2. Nach Fig. 7 ist die Pflasternachfülleinheit 3 in dem Spenderinnenraum 13 des Spendergehäuses 2 festgelegt. Das Formschloss 14 ist geschlossen und die Pflasternachfülleinheit 3 kann aus dem Spendergehäuse nicht oder nur mit einem erheblichen Aufwand entnommen werden. Hier wirkt der Formschlüssel 25 mit dem Formschloss 14 nicht zusammen. Nach Fig. 8 ist der Formschlüssel 25 in eine Führungsspur 30 eingeführt und liegt an einem Führungsabschnitt 31 des Zungenbereichs 15b beziehungsweise des zweiten Formschlossteils 14b an. Die Führungsspur 30 ist dabei an dem Außenteil 20b ausgebildet. Um den Formschlüssel 25 in die Führungsspur 30 vereinfacht einführen zu können, weist diese einen Rampenbereich 34 auf, der von dem Gehäuseboden 21 zu dem Formschloss 14 hin zuläuft. Der Rampenbereich 34 ist in Fig. 3 und in Fig. 6 angedeutet. Ferner ist in dem Gehäuseboden 21 ein weiterer Rampenbereich 35 ausgebildet, der zu dem Rampenbereich 34 gerichtet ist. Durch den Rampenbereich 35 kann der Formschlüssel 25 an dem Gehäuseboden 21 vereinfacht zu dem Rampenbereich 34 und dann durch den Rampenbereich 34 vereinfacht zu dem Formschloss 14 geführt werden. Der Führungsabschnitt 31 ist dabei unter einem Winkel zu der Entnahmerichtung 12 ausgerichtet.

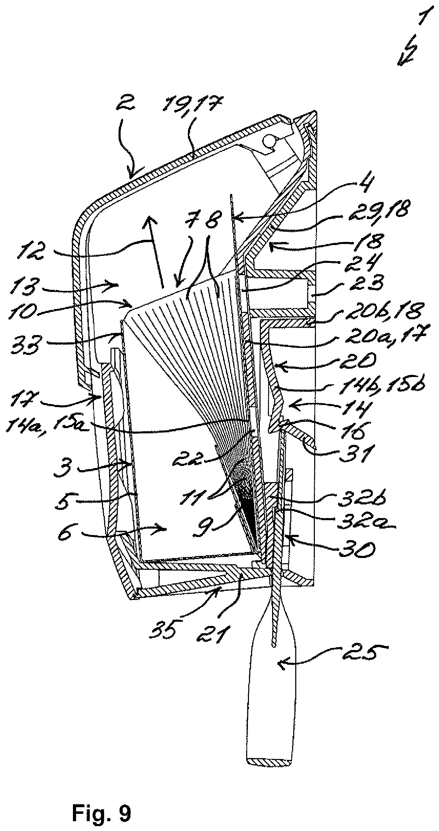

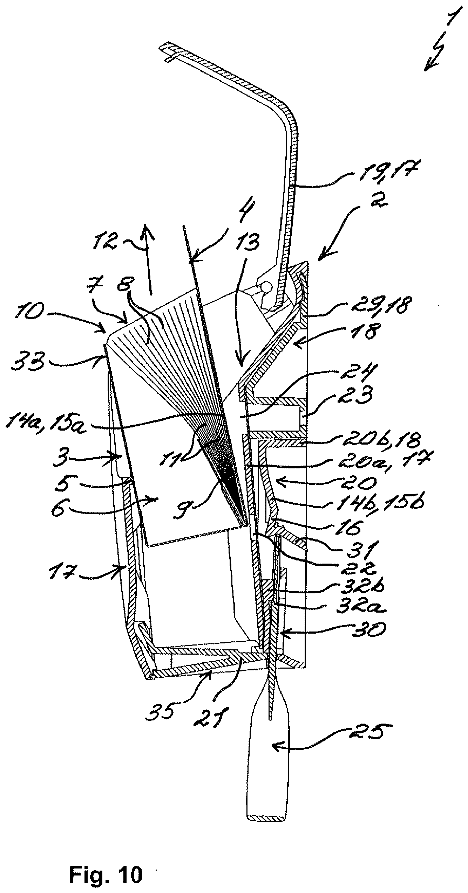

Nach Fig. 9 ist der Formschlüssel 25 in der Führungsspur 30 in Entnahmerichtung 12 geführt und wirkt mit dem Führungsabschnitt 31 des Zungenbereich 15b beziehungsweise des zweiten Formschlossteils 14b zusammen. Der Führungsabschnitt 31 gleitet dabei an dem Formschlüssel 25 quer zur Entnahmerichtung 12 ab und der Zungenbereich 15b beziehungsweise das zweite Formschlossteil 14b wird quer zur Entnahmerichtung 12 von der Eingriffsöffnung 15a beziehungsweise von dem ersten Formschlossteil 14a weggeführt. Dadurch gleitet die Eingriffsnase 16 aus der Eingriffsöffnung 15a heraus und das Formschloss 14 wird geöffnet. Ein erster Begrenzungsanschlag 32a des Formschlüssels 25 liegt dabei an einem zweiten Begrenzungsanschlag 32b des hinteren Gehäusestücks 18 an, so dass weiteres Führen des Formschlüssels 25 in der Führungsspur 30 in Entnahmerichtung 12 verhindert ist. Ist der Gehäuseboden 21 federnd ausgeführt, so kann beim Führen des Formschlüssels 25 in Entnahmerichtung 12 der Formschlüssel 25 mit dem federnden Gehäuseboden 21 zusammenwirken. Dazu kann an dem Formschlüssel 25 ein erster Hebeanschlag und an dem Gehäusebodens 21 ein zweiter Hebeanschlag ausgebildet sein, die miteinander in Entnahmerichtung 12 in Eingriff gebracht werden können. Durch das Zusammenwirken der Hebeanschläge kann dann der Gehäuseboden 21 beim Führen des Formschlüssels 25 in Entnahmerichtung 12 anheben und dadurch die Pflasternachfülleinheit 3 in dem Spenderinnenraum 13 in Entnahmerichtung 12 verschoben werden. In Fig. 10 ist der Pflasterspender 1 beim Entnehmen der Pflasternachfülleinheit 3 aus dem Spenderinnenraum 13 gezeigt.

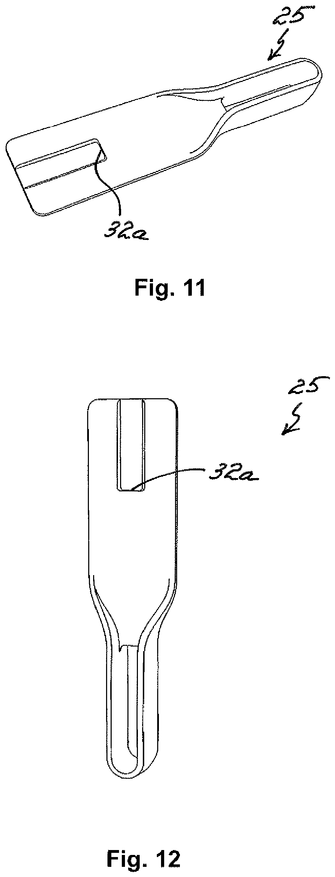

Fig. 11 und Fig. 12 zeigen Ansichten des Formschlüssels 25 zum Öffnen des Formschlosses 14.

## Patentansprüche

1. Pflasterspender (1) mit einem Spendergehäuse (2) und wenigstens einer in dem Spendergehäuse (2) angeordneten Pflasternachfülleinheit (3),
- wobei die Pflasternachfülleinheit (3) eine Aufnahmebox (4) und wenigstens ein Pflasterbündel (7) aufweist, der in einem Boxinnenraum (6) der Aufnahmebox (4) festgelegt ist und mehrere an einer offenen Entnahmeseite (10) der Aufnahmebox (4) in Entnahmerichtung (12) aus der Pflasternachfülleinheit (3) entnehmbare Einzelpflaster (8) aufweist,
- wobei die wenigstens eine Pflasternachfülleinheit (3) in einem Spenderinnenraum (13) des Spendergehäuses (2) aus diesem in Entnahmerichtung (12) entnehmbar angeordnet ist,
- wobei die wenigstens eine Pflasternachfülleinheit (3) in dem Spenderinnenraum (13) durch wenigstens ein formschlüssiges Formschloss (14) lösbar festgelegt ist, wobei ein erstes an der Aufnahmebox (4) ausgebildetes Formschlossteil (14a) und ein zweites in dem Spenderinnenraum (13) federnd angeordnetes Formschlossteil (14b) quer zur Entnahmerichtung (12) formschlüssig ineinander eingreifen, so dass die Pflasternachfülleinheit (3) aus dem Spenderinnenraum (13) beim geschlossenen Formschloss (14) nicht entnehmbar ist und beim geöffneten Formschloss (14) entnehmbar ist,
**dadurch gekennzeichnet,**
- **dass** das erste Formschlossteil (14a) durch eine Eingriffsöffnung (15a) in einer Außenwandung (5) der Aufnahmebox (4) gebildet ist und das zweite Formschlossteil (14b) ein zungenartiger und federnder Zungenbereich (15b) ist, an dem eine Eingriffsnase (16) ausgebildet ist,
- **dass** der Zungenbereich (15b) an einer den Spenderinnenraum (13) begrenzenden Spenderwand (20) des Spendergehäuses (2) ausgebildet ist und die Eingriffsnase (16) innerhalb des Spenderinnenraums (13) angeordnet ist,
- **dass** die Eingriffsnase (16) quer zur Entnahmerichtung (12) in die Eingriffsöffnung (15a) formschlüssig eingreift, und
- **dass** der Eingriff zwischen der Eingriffsnase (16) und der Eingriffsöffnung (15a) von außen ohne weitere Mittel nicht lösbar ist.

2. Pflasterspender nach Anspruch 1,
**dadurch gekennzeichnet,**
- **dass** die Spenderwand (20) ein von dem Spenderinnenraum (13) abgewandtes Außenteil (20b) und ein dem Spenderinnenraum (13) zugewandtes Innenteil (20a) aufweist,
- **dass** der Zungenbereich (15b) an dem Außenteil (20b) der Spenderwand (20) ausgebildet ist und die Eingriffsnase (16) durch eine Aussparung (22) in dem Innenteil (20a) der Spenderwand (20) in den Spenderinnenraum (13) hineinragt.

3. Pflasterspender nach einem der vorangehenden Ansprüche,
**dadurch gekennzeichnet,**
**dass** das Formschloss (14) einen Formschlüssel (25) aufweist, der den Eingriff zwischen dem ersten Formschlossteil (14a) und dem zweiten Formschlossteil (14b) von außen löst.

4. Pflasterspender nach Anspruch 3,
**dadurch gekennzeichnet,**
**dass** das zweite Formschlossteil (14b) einen Führungsabschnitt (31) aufweist, wobei beim Führen des Formschlüssels (25) in Entnahmerichtung (12) der Führungsabschnitt (31) des zweiten Formschlossteils (14b) an dem Formschlüssel (25) abgleitet und das zweite Formschlossteil (14b) quer zur Entnahmerichtung (12) von dem ersten Formschlossteil (14a) hinweg gedrückt ist, so dass der Eingriff zwischen dem zweiten Formschlossteil (14b) und dem ersten Formschlossteil (14a) gelöst und das Formschloss (14) geöffnet ist.

5. Pflasterspender nach Anspruch 3 oder 4,
**dadurch gekennzeichnet,**
**dass** an dem Spendergehäuse (2) für den Formschlüssel (25) eine dem Formschlüssel (25) komplementäre Führungsspur (30) ausgebildet ist, wobei der Formschlüssel (25) in der Führungsspur (30) in Entnahmerichtung (12) und dadurch das zweite Formschlossteil (14b) an dem Formschlüssel (25) quer zur Entnahmerichtung (12) führbar sind.

6. Pflasterspender nach einem der Ansprüche 3 bis 5,
**dadurch gekennzeichnet,**
**dass** der Spenderinnenraum (13) quer zur Entnahmerichtung (12) durch einen federnden Gehäuseboden (21) begrenzt ist, wobei beim Führen des Formschlüssels (25) in Entnahmerichtung (12) die an den Gehäuseboden (21) abgestützte Pflasternachfülleinheit (3) in Entnahmerichtung (12) aus dem Spenderinnenraum (13) verschiebbar ist.

7. Pflasterspender nach einem der vorangehenden Ansprüche,
**dadurch gekennzeichnet,**
- **dass** das Spendergehäuse (2) ein vorderes Gehäusestück (17) und ein hinteres Gehäusestück (18) aufweist, die aneinander zu dem Spendergehäuse (2) festgelegt sind, und
- **dass** das vordere Gehäusestück (17) zumindest vierseitig den Spenderinnenraum (13) begrenzt und einen schwenkbaren Gehäusedeckel (19) zum Verschließen des Spenderinnenraums (13) aufweist.

8. Pflasterspender nach Anspruch 2 und 7,
**dadurch gekennzeichnet,**
- **dass** das Innenteil (20a) der Spenderwand (20) in dem vorderen Gehäusestück (17) integral ausgebildet ist, und
- **dass** das Außenteil (20b) der Spenderwand (20) zumindest bereichsweise das hintere Gehäusestück (18) bildet und an einem Restteil (29) des hinteren Gehäusestücks (18) form- oder kraftschlüssig lösbar festgelegt ist.

9. Pflasterspender nach Anspruch 8,
**dadurch gekennzeichnet,**
- **dass** in dem hinteren Gehäusestück (18) außerhalb des Außenteils (20b) wenigstens eine Schraubenöffnung (23) ausgebildet ist, durch die hindurch das hintere Gehäusestück (18) an einer Wandung festlegbar ist, und
- **dass** die wenigstens eine Schraubenöffnung (23) mit einer Durchgangsöffnung (24) in dem Innenteil (20a) korrespondiert, wobei die Durchgangsöffnung (24) und die Schraubenöffnung (23) durch die in dem Spenderinnenraum (13) festgelegte Pflasternachfülleinheit (3) abgedeckt ist.

10. Pflasterspender nach einem der Ansprüche 7 bis 9,
**dadurch gekennzeichnet,**
- **dass** die Außenwandung (5) der Aufnahmebox (4) aus dem vorderen Gehäusestück (17) in Entnahmerichtung (12) zumindest bereichsweise herausragt, so dass ein Erkennungsbereich (33) der Außenwandung (5) von dem hinteren Gehäusestück (18) abgewandt freigelegt ist und dadurch eine in dem Erkennungsbereich (33) der Außenwandung (5) aufgebrachte Information für den Nutzer erkennbar ist, oder
- **dass** das vordere Gehäusestück (17) zumindest bereichsweise aus einem durchsichtigen Material geformt ist, so dass ein Erkennungsbereich (33) der Außenwandung (5) von dem hinteren Gehäusestück (18) abgewandt durch das vordere Gehäusestück (17) sichtbar ist und dadurch eine in dem Erkennungsbereich (33) der Außenwandung (5) aufgebrachte Information für den Nutzer erkennbar ist.

11. Pflasterspender nach einem der vorangehenden Ansprüche,
**dadurch gekennzeichnet,**
- **dass** die Außenwandung (5) quer zur Entnahmerichtung (12) einen rechteckigen Querschnitt aufweist und einseitig durch eine Abstützwand gebildet ist, und
- **dass** das Pflasterbündel (7) mit der Abstützwand der Entnahmeseite (10) gegenüberliegend verspannt ist, so dass die Einzelpflaster (8) sich zu der Entnahmeseite (10) hin quer zur Entnahmerichtung (12) auffächern.

12. Pflasterspender nach Anspruch 11,
**dadurch gekennzeichnet,**
- **dass** innerhalb des Boxinnenraums (6) eine Haltewand der Aufnahmebox (4) und die Abstützwand der Außenwandung (5) eine zu der Entnahmeseite (10) hin offene Aufnahmetasche (9) bilden, und
- **dass** das Pflasterbündel (7) in der Aufnahmetasche (9) festgelegt und mit der Haltewand und der Abstützwand verspannt ist.

13. Pflasterspender nach einem der vorangehenden Ansprüche,
**dadurch gekennzeichnet,**
**dass** die Einzelpflaster (8) des Pflasterbündels (7) an der Entnahmeseite (10) abwechselnd auf einen abweichenden Abstand hinausragen.

## Claims

1. Plaster dispenser (1) having a dispenser housing (2) and at least one plaster refill unit (3) arranged in the dispenser housing (2),
- wherein the plaster refill unit (3) has a receiving box (4) and at least one plaster bundle (7) which is fixed in a box interior (6) of the receiving box (4) and has several individual plasters (8) which are removable from the plaster refill unit (3) at an open removal side (10) of the receiving box (4) in removal direction (12),
- wherein the at least one plaster refill unit (3) is arranged in a dispenser interior (13) of the dispenser housing (2) in a way so as to be removable therefrom in removal direction (12),
- wherein the at least one plaster refill unit (3) is releasably fixed in the dispenser interior (13) by means of at least one form-fitting form lock (14), wherein a first form lock part (14a) formed on the receiving box (4) and a second form lock part (14b) arranged resiliently in the dispenser interior (13) engage form-fittingly with one another transversely to the removal direction (12) such that the plaster refill unit (3) cannot be removed from the dispenser interior (13) when the form lock (14) is closed and can be removed when the form lock (14) is open,
**characterised in that**
- the first form lock part (14a) is formed by an engagement opening (15a) in an outer wall (5) of the receiving box (4) and the second form lock part (14b) is a tongue-like and resilient tongue region (15b) on which an engagement nose (16) is formed,
- the tongue region (15b) is formed on a dispenser wall (20) of the dispenser housing (2) delimiting the dispenser interior (13) and the engagement nose (16) is arranged within the dispenser interior (13),
- the engagement nose (16) engages form-fittingly in the engagement opening (15a) transversely to the removal direction (12), and
- the engagement between the engagement nose (16) and the engagement opening (15a) is not releasable from the outside without further means.

2. Plaster dispenser according to claim 1,
**characterised in that**
- the dispenser wall (20) has an outer part (20b) facing away from the dispenser interior (13) and an inner part (20a) facing the dispenser interior (13),
- the tongue region (15b) is formed on the outer part (20b) of the dispenser wall (20) and the engagement nose (16) protrudes through a cutout (22) in the inner part (20a) of the dispenser wall (20) into the dispenser interior (13).

3. Plaster dispenser according to any of the preceding claims,
**characterised in that**
the form lock (14) has a shaped key (25) which releases the engagement between the first form lock part (14a) and the second form lock part (14b) from the outside.

4. Plaster dispenser according to claim 3,
**characterised in that**
the second form lock part (14b) has a guide section (31), wherein when the shaped key (25) is guided in removal direction (12) the guide section (31) of the second form lock part (14b) slides off the shaped key (25) and the second form lock part (14b) is pressed away from the first form lock part (14a) transversely to the removal direction (12) such that the engagement between the second form lock part (14b) and the first form lock part (14a) is released and the form lock (14) is opened.

5. Plaster dispenser according to claim 3 or 4,
**characterised in that**
a guide track (30) complementary to the shaped key (25) is formed on the dispenser housing (2) for the shaped key (25), wherein the shaped key (25) can be guided in the guide track (30) in removal direction (12) and as a result the second form lock part (14b) can be guided on the shaped key (25) transversely to the removal direction (12).

6. Plaster dispenser according to any of claims 3 to 5,
**characterised in that**
the dispenser interior (13) is delimited transversely to the removal direction (12) via a resilient housing base (21), wherein when the shaped key (25) is guided in removal direction (12) the plaster refill unit (3) supported on the housing base (21) is displaceable in removal direction (12) out of the dispenser interior (13).

7. Plaster dispenser according to any of the preceding claims,
**characterised in that**
- the dispenser housing (2) has a front housing piece (17) and a rear housing piece (18) which are fixed to the dispenser housing (2) against one another, and
- the front housing piece (17) delimits the dispenser interior (13) at least on four sides and has a housing lid (19) for closing the dispenser interior (13).

8. Plaster dispenser according to claim 2 and 7,
**characterised in that**
- the inner part (20a) of the dispenser wall (20) is formed integrally in the front housing piece (17), and
- the outer part (20b) of the dispenser wall (20) forms at least in some sections the rear housing piece (18) and is fixed releasably in a form-fitting or force-fitting manner to a residual part (29) of the rear housing piece (18).

9. Plaster dispenser according to claim 8,
**characterised in that**
- in the rear housing piece (18) externally to the outer part (20b) is formed at least one screw opening (23) through which the rear housing piece (18) can be fixed to a wall, and
- the at least one screw opening (23) corresponds with a through opening (24) in the inner part (20a), wherein the through opening (24) and the screw opening (23) are covered by the plaster refill unit (3) fixed in the dispenser interior (13).

10. Plaster dispenser according to any of claims 7 to 9,
**characterised in that**
- the outer wall (5) of the receiving box (4) protrudes out of the front housing piece (17) in removal direction (12) at least in some regions, such that a recognition region (33) of the outer wall (5) is revealed facing away from the rear housing piece (18) and thus an item of information mounted in the recognition region (33) of the outer wall (5) is discernible for the user, or
- the front housing piece (17) is formed at least in some regions of a transparent material, such that a recognition region (33) of the outer wall (5) facing away from the rear housing piece (18) is visible through the front housing piece (17) and thus an item of information mounted in the recognition region (33) of the outer wall (5) is discernible for the user.

11. Plaster dispenser according to any of the preceding claims,
**characterised in that**
- the outer wall (5) has a rectangular cross-section transversely to the removal direction (12) and is formed on one side by a support wall, and
- the plaster bundle (7) is tensioned with the support wall opposite to the removal side (10) such that the individual plasters (8) fan out towards the removal side (10) transversely to the removal direction (12).

12. Plaster dispenser according to claim 11,
**characterised in that**
- within the box interior (6) a holding wall of the receiving box (4) and the support wall of the outer wall (5) form a receiving pocket (9) open towards the removal side (10), and
- the plaster bundle (7) is fixed in the receiving pocket (9) and is tensioned with the holding wall and the support wall.

13. Plaster dispenser according to any of the preceding claims,
**characterised in that**
the individual plasters (8) of the plaster bundle (7) protrudes out on the removal side (10) alternatingly with a deviating spacing.

## Revendications

1. Distributeur de pansement (1) avec un boîtier de distributeur (2) et au moins une unité de remplissage de pansement (3) agencée dans le boîtier de distributeur (2),
- dans lequel l'unité de remplissage de pansement (3) présente une boîte de réception (4) et au moins un paquet de pansement (7) qui est fixé dans un espace intérieur de boîte (6) de la boîte de réception (4) et présente plusieurs pansements individuels (8) retirables de l'unité de remplissage de pansement (3) au niveau d'un côté de retrait (10) ouvert de la boîte de réception (4) dans le sens de retrait (12),
- dans lequel l'au moins une unité de remplissage de pansement (3) est agencée de manière retirable dans un espace intérieur de distributeur (13) du boîtier de distributeur (2) de celui-ci dans le sens de retrait (12),
- dans lequel l'au moins une unité de remplissage de pansement (3) est fixée de manière détachable dans l'espace intérieur de distributeur (13) par au moins une fermeture de formes (14) à complémentarité de formes, dans lequel une première partie de fermeture de formes (14a) réalisée au niveau de la boîte de réception (4) et une seconde partie de fermeture de formes (14b) agencée de manière élastique dans l'espace intérieur de distributeur (13) s'engagent l'une dans l'autre par complémentarité de formes transversalement au sens de retrait (12), de sorte que l'unité de remplissage de pansement (3) ne puisse être retirée de l'espace intérieur de distributeur (13) en cas de fermeture de formes (14) fermée et puisse être retirée en cas de fermeture de formes (14) ouverte,
**caractérisé en ce que**
- la première partie de fermeture de formes (14a) est formée par une ouverture d'engagement (15a) dans une paroi extérieure (5) de la boîte de réception (4) et la seconde partie de fermeture de formes (14b) est une zone de languette (15b) à languette et à ressort, au niveau de laquelle un nez d'engagement (16) est réalisé,
- la zone de languette (15b) est réalisée au niveau d'une paroi de distributeur (20) délimitant l'espace intérieur de distributeur (13) du boîtier de distributeur (2) et le nez d'engagement (16) est agencé à l'intérieur de l'espace intérieur de distributeur (13),
- le nez d'engagement (16) s'engage par complémentarité de formes transversalement au sens de retrait (12) dans l'ouverture d'engagement (15a), et
- l'engagement entre le nez d'engagement (16) et l'ouverture d'engagement (15a) n'est pas détachable de l'extérieur sans autre moyen.

2. Distributeur de pansement selon la revendication 1,
**caractérisé en ce que**
- la paroi de distributeur (20) présente une partie extérieure (20b) éloignée de l'espace intérieur de distributeur (13) et une partie intérieure (20a) tournée vers l'espace intérieur de distributeur (13),
- la zone de languette (15b) est réalisée au niveau de la partie extérieure (20b) de la paroi de distributeur (20) et le nez d'engagement (16) pénètre par un évidement (22) dans la partie intérieure (20a) de la paroi de distributeur (20) dans l'espace intérieur de distributeur (13).

3. Distributeur de pansement selon l'une quelconque des revendications précédentes,
**caractérisé en ce que**
la fermeture de formes (14) présente une clé de formes (25) qui détache l'engagement entre la première partie de fermeture de formes (14a) et la seconde partie de fermeture de formes (14b) de l'extérieur.

4. Distributeur de pansement selon la revendication 3,
**caractérisé en ce que**
la seconde partie de fermeture de formes (14b) présente une section de guidage (31), dans lequel lors du guidage de la clé de formes (25) dans le sens de retrait (12), la section de guidage (31) de la seconde partie de fermeture de formes (14b) glisse sur la clé de formes (25) et la seconde partie de fermeture de formes (14b) est pressée transversalement au sens de retrait (12) de la première partie de fermeture de formes (14a), de sorte que l'engagement entre la seconde partie de fermeture de formes (14b) et la première partie de fermeture de formes (14a) soit détaché et la fermeture de formes (14) soit ouverte.

5. Distributeur de pansement selon la revendication 3 ou 4,
**caractérisé en ce que**
une voie de guidage (30) complémentaire à la clé de formes (25) est réalisée au niveau du boîtier de distributeur (2) pour la clé de formes (25), dans lequel la clé de formes (25) peut être guidée dans la voie de guidage (30) dans le sens de retrait (12) et ainsi la seconde partie de fermeture de formes (14b) peut être guidée au niveau de la clé de formes (25) transversalement au sens de retrait (12).

6. Distributeur de pansement selon l'une quelconque des revendications 3 à 5,
**caractérisé en ce que**
l'espace intérieur de distributeur (13) est délimité transversalement au sens de retrait (12) par un fond de boîtier (21) à ressort, dans lequel lors du guidage de la clé de formes (25) dans le sens de retrait (12), l'unité de remplissage de pansement (3) en appui contre le fond de boîtier (21) est coulissante dans le sens de retrait (12) de l'espace intérieur de distributeur (13).

7. Distributeur de pansement selon l'une quelconque des revendications précédentes,
**caractérisé en ce que**
- le boîtier de distributeur (2) présente une pièce de boîtier avant (17) et une pièce de boîtier arrière (18) qui sont fixées l'une à l'autre pour former le boîtier de distributeur (2), et
- la pièce de boîtier avant (17) délimite au moins sur les quatre côtés l'espace intérieur de distributeur (13) et présente un couvercle de boîtier (19) pivotant pour la fermeture de l'espace intérieur de distributeur (13).

8. Distributeur de pansement selon les revendications 2 et 7,
**caractérisé en ce que**
- la partie intérieure (20a) de la paroi de distributeur (20) est réalisée d'un seul tenant dans la pièce de boîtier avant (17), et
- la partie extérieure (20b) de la paroi de distributeur (20) forme au moins par endroits la pièce de boîtier arrière (18) et est fixée de manière détachable à une partie restante (29) de la pièce de boîtier arrière (18) par complémentarité de formes ou à force.

9. Distributeur de pansement selon la revendication 8,
**caractérisé en ce que**
- au moins une ouverture de vis (23) est réalisée dans la pièce de boîtier arrière (18) en dehors de la partie extérieure (20b), à travers laquelle la pièce de boîtier arrière (18) peut être fixée à une paroi, et
- l'au moins une ouverture de vis (23) correspond à une ouverture débouchante (24) dans la partie intérieure (20a), dans lequel l'ouverture débouchante (24) et l'ouverture de vis (23) sont recouvertes par l'unité de remplissage de pansement (3) fixée dans l'espace intérieur de distributeur (13).

10. Distributeur de pansement selon l'une quelconque des revendications 7 à 9,
**caractérisé en ce que**
- la paroi extérieure (5) de la boîte de réception (4) dépasse de la pièce de boîtier avant (17) dans le sens de retrait (12) au moins par endroits, de sorte qu'une zone de reconnaissance (33) de la paroi extérieure (5) soit exposée éloignée de la pièce de boîtier arrière (18) et ainsi une information appliquée dans la zone de reconnaissance (33) de la paroi extérieure (5) est reconnaissable pour l'utilisateur, ou
- la pièce de boîtier avant (17) est formée au moins par endroits en un matériau transparent, de sorte qu'une zone de reconnaissance (33) de la paroi extérieure (5) soit visible éloignée de la pièce de boîtier arrière (18) à travers la pièce de boîtier avant (17) et ainsi une information appliquée dans la zone de reconnaissance (33) de la paroi extérieure (5) est reconnaissable pour l'utilisateur.

11. Distributeur de pansement selon l'une quelconque des revendications précédentes,
**caractérisé en ce que**
- la paroi extérieure (5) présente transversalement au sens de retrait (12) une section transversale rectangulaire et est formée d'un côté par une paroi d'appui, et
- le paquet de pansement (7) est serré avec la paroi d'appui à l'opposé du côté de retrait (10) de sorte que les pansements individuels (8) se déploient vers le côté de retrait (10) transversalement au sens de retrait (12).

12. Distributeur de pansement selon la revendication 11,
**caractérisé en ce que**
- une paroi de retenue de la boîte de réception (4) et la paroi d'appui de la paroi extérieure (5) forment dans l'espace intérieur de boîte (6) une poche de réception (9) ouverte vers le côté de retrait (10), et
- le paquet de pansement (7) est fixé dans la poche de réception (9) et est serré avec la paroi de retenue et la paroi d'appui.

13. Distributeur de pansement selon l'une quelconque des revendications précédentes,
**caractérisé en ce que**
les pansements individuels (8) du paquet de pansement (7) dépassent au niveau du côté de retrait (10) en alternance à une distance différente.
